# EUROPEAN PATENT APPLICATION

(11) **EP 2 422 880 A2**
(43) Date of publication of application: **29.02.2012**
(21) Application number: 11176572.3
(22) Date of filing: 04.08.2011
(51) Int. Cl.: B01L 9/06, B01L 3/00

(54) **Container for vials**

(30) Priority: 30.08.2010 US 378010 P; 29.06.2011 US 172087
(71) Applicant: Agilent Technologies, Inc., Santa Clara, CA 95051 (US)
(72) Inventor: Whitehead, Lance, Loveland, CO Colorado 80537-0599 (US)
(74) Representative: Barth, Daniel Mathias

(57) **Abstract**

A container (10) for holding vials for use with automated analysis devices has a bottom (12) and four sidewalls (14,16,18,20). The sidewalls (14,16,18,20) extend perpendicularly to the bottom (12). Two of the sidewalls are oppositely disposed from one another and have corrugations (22) formed by crests (24) and troughs (26). The corrugations (22) are arranged such that the crests (24) on one sidewall align with the troughs (26) on the opposite sidewall. Each trough (26) has three angularly oriented panels (28,30,32) and is sized to receive and support a vial through contact with only two of the three panels. A cover (44) is also provided. The cover (44) has a top (46) and four sidewalls (48,50,52,54) which extend perpendicularly to the top (46). Two of the top's sidewalls are oppositely disposed from one another and have corrugations which match and align with the corrugations (56) of the container (10). A skirt is attached to the sidewalls of the cover (44) and forms a perimeter which receives the container (10).

## Description

### Cross Reference to Related Application

This application is based upon and claims priority to U.S. Provisional Application No. 61/378,010, filed August 30, 2010 and which application is hereby incorporated in its entirely herein

### Field of the Invention

This invention concerns containers for holding, storing, transporting, organizing and protecting vials which hold samples for analysis.

### Background

Glass vials are used to convey samples to automated analysis devices such as chromatographs and mass spectrometers. To ensure proper and efficient functioning of such automated devices it is desirable to house the vials in a container having a size that is compatible with the analysis device and which organizes and presents the vials for easy access by the robotic components which handle them. It is also desirable that the container protect the vials against contamination as well as breakage, as can occur, for example, when the container is accidently dropped onto a hard surface.

### Brief Description of the Drawings

Figure 1 is an isometric view of an example embodiment of a container according to the invention;
Figure 2 is a top plan view of the container shown in Figure 1;
Figure 3 is an isometric view of an example embodiment of a cover for the container shown in Figure 1;
Figure 4 is a top plan view of the cover shown in Figure 3;
Figure 5 is an isometric view of the container shown in Figure 1 and the cover shown in Figure 3;
Figure 6 is an isometric view of an example container holding a plurality of vials; and
Figure 7 is a partial plan view of the container shown in Figure 6.

### Detailed Description

The invention concerns a container for holding a plurality of vials in a plurality of adjacent rows. The container comprises a bottom for supporting the vials and first and second oppositely disposed sidewalls attached to the bottom. The sidewalls extend perpendicularly to the bottom. Each of the sidewalls has corrugations formed of a plurality of alternating crests and troughs. Each of the troughs comprises a first trough panel, a second trough panel and a third trough panel. Each of the trough panels extend perpendicularly to the bottom and are oriented angularly with respect to one another. Each of the troughs are sized to receive and support one of the vials between the first trough panel and the third trough panel. For each of the troughs, the first and third trough panels are arranged on opposite sides of the second trough panel. The container also includes third and fourth oppositely disposed sidewalls attached to the bottom. The third and fourth sidewalls extend perpendicularly to the bottom and are in spaced relation to one another. The third and fourth sidewalls extend between the first and second sidewalls.

In one embodiment, the troughs of the first sidewall are aligned with the crests of the second sidewall. In this configuration each one of the rows of vials within the container extend between one of the troughs of the first sidewall and one of the crests of the second sidewall.

In one embodiment, for each of the troughs, the first trough panel may be oriented at an orientation angle from about 100° to about 140° with respect to the second trough panel; for example, orientation angles such as about 100°, 105°, 110°, 115°, 120°, 125°, 130°, 135°, or 140° are feasible. Additionally, for each of the troughs, the third trough panel may be oriented at an orientation angle from about 100° to about 140° with respect to the second trough panel; for example, orientation angles such as about 100°, 105°, 110°, 115°, 120°, 125°, 130°, 135°, or 140° are feasible.

In one embodiment, each of the crests comprises a first crest panel, a second crest panel and a third crest panel extending perpendicularly to the bottom. The crest panels are oriented angularly with respect to one another. For each of the crests, the first and third crest panels are arranged on opposite sides of the second crest panel. For each of the crests, the first crest panel may be oriented at an orientation angle from about 100° to about 140° with respect to the second crest panel; for example, orientation angles such as about 100°, 105°, 110°, 115°, 120°, 125°, 130°, 135°, or 140° are feasible. Furthermore, for each of the crests, the third crest panel may be oriented at an orientation angle from about 100° to about 140° with respect to the second crest panel; for example, orientation angles such as about 100°, 105°, 110°, 115°, 120°, 125°, 130°, 135°, or 140° are feasible.

The container may also comprise a cover. In one embodiment, the cover comprises a top positionable overlying and in spaced relation to the bottom. The cover has first and second oppositely disposed cover sidewalls attached to the top and extending perpendicularly thereto. Each of the cover sidewalls has corrugations formed of a plurality of alternating crests and troughs. Each of the troughs of the cover comprise a first cover trough panel, a second cover trough panel and a third cover trough panel. Each of the cover trough panels extend perpendicularly to the top and are oriented angularly with respect to one another. Each of the troughs of the cover may be the same size as the troughs of the container. For each of the troughs of the cover, the first and third cover trough panels are arranged on opposite sides of the second cover trough panel. The troughs of the cover align with the troughs of the container when the top is positioned in overlying spaced relation with the bottom. The cover also includes third and fourth oppositely disposed cover sidewalls which are attached to the top and extend perpendicularly thereto. The third and fourth cover sidewalls are in spaced relation to one another and extending between the first and second cover sidewalls.

In one embodiment the cover further comprises a skirt attached to the cover sidewalls. The skirt extends perpendicularly to the top and forms a perimeter having an inwardly facing surface sized to receive the container when the top is positioned in overlying spaced relation with the bottom.

In one embodiment, for each of the troughs of the cover, the first cover trough panel may be oriented at an orientation angle from about 100° to about 140° with respect to the second cover trough panel; for example, orientation angles such as about 100°, 105°, 110°, 115°, 120°, 125°, 130°, 135°, or 140° are feasible. Furthermore, for each of the troughs of the cover, the third cover trough panel may be oriented at an orientation angle from about 100° to about 140° with respect to the second cover trough panel; for example, orientation angles such as about 100°, 105°, 110°, 115°, 120°, 125°, 130°, 135°, or 140° are feasible.

Additionally, each of the crests of the cover may comprise a first cover crest panel, a second cover crest panel and a third cover crest panel extending perpendicularly to the top and oriented angularly with respect to one another. For each of the crests of the cover, the first and third cover crest panels are arranged on opposite sides of the second cover crest panel. For each of the crests of the cover, the first cover crest panel may be oriented at an orientation angle from about 100° to about 140° with respect to the second cover crest panel; for example, orientation angles such as about 100°, 105°, 110°, 115°, 120°, 125°, 130°, 135°, or 140° are feasible. Furthermore, for each of the crests of the cover, the third cover crest panel may be oriented at an orientation angle from about 100° to about 140° with respect to the second cover crest panel; for example, orientation angles such as about 100°, 105°, 110°, 115°, 120°, 125°, 130°, 135°, or 140° are feasible.

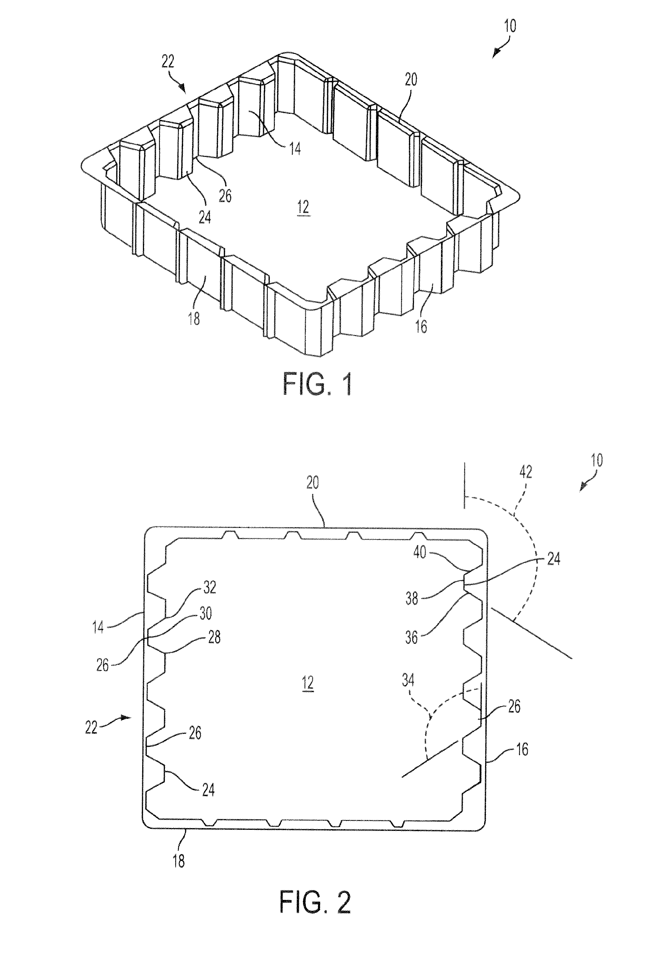

Figure 1 shows an example embodiment of a container 10 for holding a plurality of vials (not shown). Container 10 comprises a bottom 12 for supporting the vials. First and second sidewalls 14 and 16 are attached to the bottom and extend generally perpendicular to it. Sidewalls 14 and 16 are oppositely disposed from one another and in spaced apart relation. Third and fourth sidewalls 18 and 20 are also attached to the bottom 12 and extend generally perpendicular to it. The sidewalls 18 and 20 are oppositely disposed from one another in spaced apart relation and extend between the sidewalls 14 and 16.

Sidewalls 14 and 16 have corrugations 22 formed of a plurality of crests 24 and troughs 26. As shown in Figure 2, each of the troughs 26 comprises three trough panels including a flank or first trough panel 28, a center or second trough panel 30 and another flank or third trough panel 32. Panels 28, 30 and 32 extend perpendicularly to the bottom 12 and are oriented angularly to each other. Flank panels 28 and 32 are arranged on opposite sides of center panel 30 and, in this example embodiment, are oriented at an orientation angle 34 of about 120° relative to the center panel 30. This orientation angle is thought to be advantageous for a 2ml vial. Orientation angles 34 from about 100° to about 140°, for example, orientation angles such as about 100°, 105°, 110°, 115°, 120°, 125°, 130°, 135°, or 140° are feasible. Troughs 26 are sized in relation to the vials to be stored in the container 10 such that the flank panels 28 and 32 can receive and support a vial without contact between the vial and the center panel 30 as described in detail below.

As further shown in Figure 2, the crests 24 may also be described as comprising three crest panels including a flank or first crest panel 36, a center or second crest panel 38 and another flank or third crest panel 40. The crest panels extend perpendicularly to the bottom 12 and are oriented angularly with respect to one another. In this example embodiment, the crest flank panels 36 and 40 are positioned on opposite sides of the center crest panel 38 and are oriented at an orientation angle 42 of about 120° with respect to it. Orientation angles 42 from about 100° to about 140°, for example, orientation angles such as about 100°, 105°, 110°, 115°, 120°, 125°, 130°, 135°, or 140° are feasible. In the embodiment shown in the figures, the crests and troughs are arranged such that the troughs of sidewall 14 align with the crests of sidewall 16, and the troughs of sidewall 16 align with the crests of sidewall 14 whereby a row of vials positioned on bottom 12 would extend from a trough 26 in sidewall 14 to a crest 24 in sidewall 16 and vice versa. Other alignment relationships between the crests and troughs are also feasible, for example, the crests of sidewall 14 aligning with the crests of sidewall 16 and the troughs of sidewall 14 aligning with the troughs of sidewall 16.

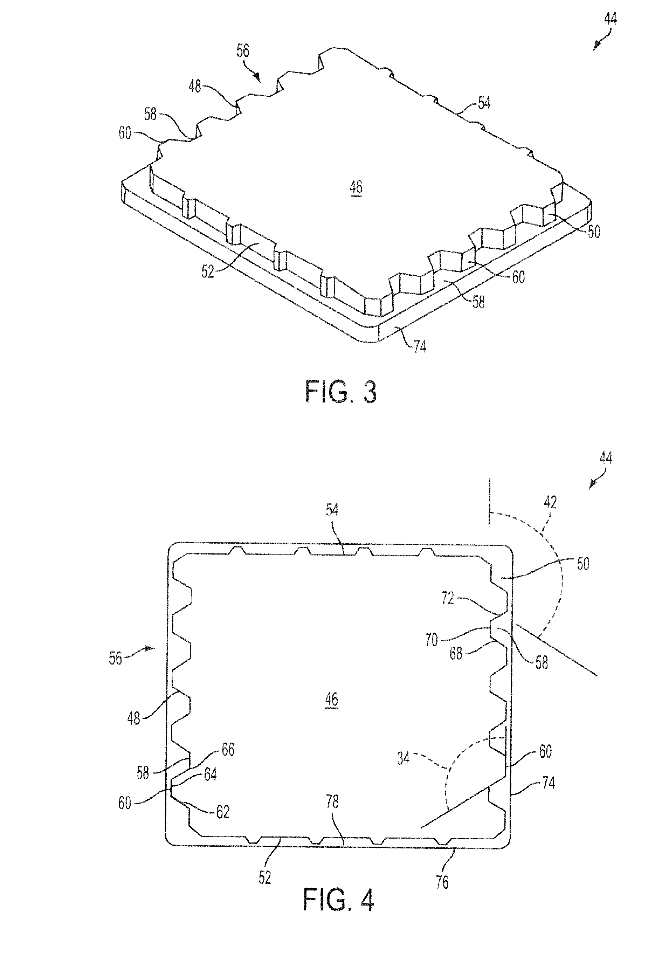

The container 10 may also include a cover 44, shown in Figure 3. Cover 44 comprises a top 46 to which are attached two sidewalls 48 and 50. Sidewalls 48 and 50 are oppositely disposed in spaced apart relation and extend generally perpendicularly to the cover. Two additional sidewalls 52 and 54 (third and fourth cover sidewalls) are also attached to the top 44 and extend generally perpendicular to it. The sidewalls 52 and 54 are oppositely disposed from one another in spaced apart relation and extend between the sidewalls 48 and 50.

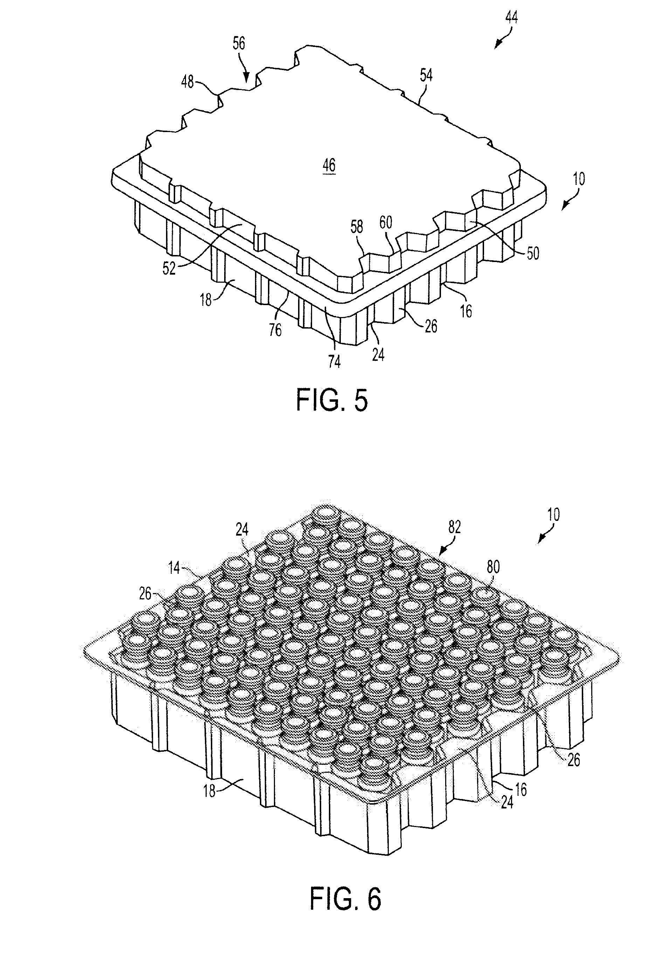

Sidewalls 48 and 50 have corrugations 56 also formed of a plurality of crests 58 and troughs 60. As shown in Figure 4, each of the troughs 60 comprises three trough panels including a flank or first trough panel 62, a center or second trough panel 64 and another flank or third trough panel 66. Panels 62, 64 and 66 extend perpendicularly to the top 46 and are oriented angularly to each other. Flank panels 62 and 66 are arranged on opposite sides of center panel 64 and, in this example embodiment, are oriented at the same orientation angle 34 of about 120° relative to the center panel 64 as the panels comprising the troughs 26 of the container 10. Orientation angles 34 from about 100° to about 140°, for example, orientation angles such as about 100°, 105°, 110°, 115°, 120°, 125°, 130°, 135°, or 140° are feasible. Troughs 60 of the cover 44 are sized and positioned to match and align with the troughs 26 of the container 10 when the cover is placed with its top 46 in overlying relation with bottom 12 as shown in Figure 5.

As further shown in Figure 4, the crests 58 of the cover 44 may also be described as comprising three crest panels including a flank or first crest panel 68, a center or second crest panel 70 and another flank or third crest panel 72. The crest panels extend perpendicularly to the top 46 and are oriented angularly with respect to one another. In this example embodiment the crest flank panels 68 and 72 are positioned on opposite sides of the center crest panel 70 and are oriented at the same orientation angle 42 of about 120° relative to the center panel 70 as the panels comprising the crests 24 of the container 10. Orientation angles from about 100° to about 140°, for example, orientation angles such as about 100°, 105°, 110°, 115°, 120°, 125°, 130°, 135°, or 140° are feasible. Crests 58 of the cover 44 are sized and positioned to match and align with the crests 24 of the container 10 when the cover is placed with its top 46 in overlying relation with bottom 12 as shown in Figure 5.

As shown in Figure 3, cover 44 may also comprise a skirt 74. Skirt 74 is attached to the cover sidewalls 48, 50, 52 and 54 and extends generally perpendicular to the top 46. As shown in Figure 4, the skirt 74 forms a perimeter 76 which has an inwardly facing surface 78. The surface 78 is sized to receive the container 10 when top 46 is positioned in overlying spaced relation to the bottom 12 as shown in Figure 5.

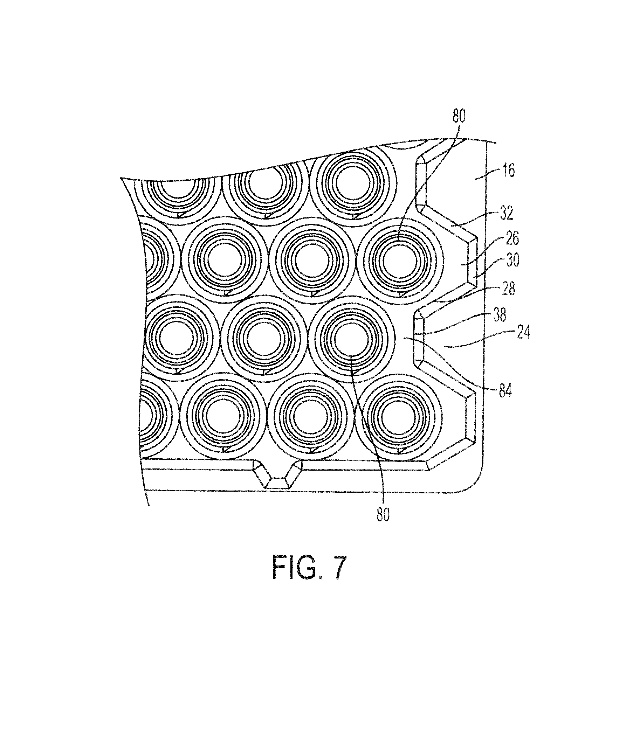

Figure 6 illustrates the container embodiment 10 holding a plurality of vials 80 arranged in adjacent rows 82. Note that for this container embodiment, each row 82 of vials 80 extends from a trough 26 on one sidewall (14 or 16) to a crest 24 on the opposite sidewall. Figure 7 illustrates in detail the interaction between the vials 80 and the sidewalls 14 and 16 to highlight the features which are expected to provide protection against breakage of the vials when they are subjected to impact forces, as for example, when the container is dropped. As noted above, each trough 26 is sized to receive a vial 80 such that the vial will only contact the flank panels 28 and 32. This helps reduce the transmission of impact force to the vial to avoid breakage. However, where a vial 80 is adjacent to a crest 24, the staggered arrangement of the vials (due to the alignment of the crests on one sidewall with the troughs on the opposite sidewall) permits a gap 84 to exist between the crest's center panel 38 and the adjacent vial 80, thereby isolating the vial from direct impact loads which would otherwise be transmitted through the sidewall.

It is advantageous to make the container 10 and cover 44 from polymer materials such as polyethylene, polyethylene terephthalate, or polyvinyl chloride (PVC). PVC is expected to provide additional protection due to its relatively high impact resistance when compared with other polymers. Drop tests performed on a container and cover according to the invention have shown an average reduction in broken vials of 40% as compared with the same tests performed on a container made of the same material but having sides without corrugations. It is expected that containers according to the invention may be employed to hold glass vials from about 1ml to about 20ml volume with decreased chance of breakage resulting from dropped containers.

## Claims

1. A container (10) for holding a plurality of vials (80) in a plurality of adjacent rows (82), said container (10) comprising:
a bottom (12) for supporting said vials (80);
first and second oppositely disposed sidewalls (14, 16) attached to said bottom (12) and extending perpendicularly thereto, each of said sidewalls (14, 16) having corrugations (22) formed of a plurality of alternating crests (24) and troughs (26), each of said troughs comprising a first trough panel (28), a second trough panel (30) and a third trough panel (32), each of said trough panels (28, 30, 32) extending perpendicularly to said bottom (12) and oriented angularly with respect to one another, each of said troughs (26) being sized to receive and support one of said vials (80) between said first trough panel (28) and said third trough panel (32), for each of said troughs (26), said first and third trough panels (28, 32) are arranged on opposite sides of said second trough panel (30),
third and fourth oppositely disposed sidewalls (18, 20) attached to said bottom (12) and extending perpendicularly thereto, said third and fourth sidewalls (18, 20) being in spaced relation to one another and extending between said first and second sidewalls (14, 16).

2. The container (10) according to Claim 1, wherein said troughs (26) of said first sidewall (14) are aligned with said crests (24) of said second sidewall (16), whereby each one of said rows (82) extend between one of said troughs (26) of said first sidewall (14) and one of said crests (24) of said second sidewall (16).

3. The container (10) according to Claim 1 or 2, wherein, for each of said troughs (26), said first and third trough panels (28, 32) are oriented at an orientation angle (34) from about 100° to about 140°, preferably from about 115° to about 125° and most preferably of about 120°, with respect to said second trough panel (30).

4. The container (10) according to any one of Claims 1 to 3, wherein each of said crests (24) comprises a first crest panel (36), a second crest panel (38) and a third crest panel (40) extending perpendicularly to said bottom (12) and oriented angularly with respect to one another, for each of said crests (24), said first and third crest panels (36, 40) are arranged on opposite sides of said second crest panel (38).

5. The container according (10) to Claim 4, wherein, for each of said crests (24), said first and third crest panels (36, 40) are oriented at an orientation angle (42) from about 100° to about 140°, preferably from about 115° to about 125° and most preferably about 120°, with respect to said second crest panel (38).

6. The container (10) according to any one of Claim 1 to 5, further comprising a cover (44), said cover (44) comprising:
a top (46) positionable overlying and in spaced relation to said bottom (12);
first and second oppositely disposed cover sidewalls (48, 50) attached to said top (46) and extending perpendicularly thereto, each of said cover sidewalls (48, 50) having corrugations (56) formed of a plurality of alternating crests (58) and troughs (60), each of said troughs (60) of said cover (44) comprising a first cover trough panel (62), a second cover trough panel (64) and a third cover trough panel (66), each of said cover trough panels (62, 64, 66) extending perpendicularly to said top (46) and being oriented angularly with respect to one another, each of said troughs (60) of said cover (44) being the same size as said troughs (26) of said container (10), for each of said troughs (60) of said cover (44), said first and third cover trough panels (62, 66) are arranged on opposite sides of said second cover trough panel (64), said troughs (60) of said cover (44) aligning with said troughs (26) of said container (10) when said top (46) is positioned in overlying spaced relation with said bottom (12);
third and fourth oppositely disposed cover sidewalls (52, 54) attached to said top (46) and extending perpendicularly thereto, said third and fourth cover sidewalls (52, 54) being in spaced relation to one another and extending between said first and second cover sidewalls (48, 50).

7. The container (10) according to Claim 6, wherein said troughs (60) of said first cover sidewall (48) are aligned with said crests (58) of said second cover sidewall (50).

8. The container (10) according to Claim 6 or 7, further comprising a skirt (74) attached to said cover sidewalls (48, 50, 52, 54) and extending perpendicularly to said top (46), said skirt (74) forming a perimeter (76) having an inwardly facing surface (78) sized to receive said container (10) when said top (46) is positioned in overlying spaced relation with said bottom (12).

9. The container (10) according to any one of Claims 6 to 8, wherein, for each of said troughs (60) of said cover (44), said first and third cover trough panels (62, 66) are oriented at an orientation angle (34) from about 100° to about 140°, preferably from about 115° to about 125° and most preferably of about 120°, with respect to said second cover trough panel (64).

10. The container according to any one of Claims 6 to 9, wherein each of said crests (58) of said cover (44) comprises a first cover crest panel (68), a second cover crest panel (70) and a third cover crest panel (72) extending perpendicularly to said top (46) and oriented angularly with respect to one another, for each of said crests (58) of said cover (44), said first and third cover crest panels (68, 72) are arranged on opposite sides of said second cover crest panel (70).

11. The container (10) according to Claim 10, wherein, for each of said crests (58) of said cover (44), said first and third cover crest panels (68, 72) are oriented at an orientation angle (42) from about 100° to about 140°, preferably from about 115° to about 125° and most preferably of about 120°, with respect to said second cover crest panel (70).

12. The container (10) according to any one of Claims 1 to 11, further comprising a plurality of vials (80) supported on said bottom (12) and arranged in a plurality of rows (82).

13. The container (10) according to Claim 12, wherein each trough (26) of the bottom (12) is sized to receive a vial (80) such that the vial (80) only contacts the first and the third trough panel (28, 32).

14. The container (10) according to Claim 12 or 13, wherein, when a vial (80) adjacent to a crest of the bottom (12), a gap (84) exists between the crest's (24) second crest panel (38) and the adjacent vial (80).
